Europäisches Patentamt

European Patent Office

Office européen des brevets

(11) Numéro de publication: **0 237 442 B1**

(12) **FASCICULE DE BREVET EUROPEEN**

(45) Date de publication de fascicule du brevet:
**12.02.92**

(51) Int. Cl.⁵: **C13K 1/00**, C12P 19/24

(21) Numéro de dépôt: **87400568.9**

(22) Date de dépôt: **13.03.87**

Le dossier contient des informations techniques présentées postérieurement au dépôt de la demande et ne figurant pas dans le présent fascicule.

(54) **Procédé de fabrication de dextrose cristallisé à partir de saccharose et installations pour sa mise en oeuvre.**

(30) Priorité: **14.03.86 FR 8603688**

(43) Date de publication de la demande:
**16.09.87 Bulletin 87/38**

(45) Mention de la délivrance du brevet:
**12.02.92 Bulletin 92/07**

(84) Etats contractants désignés:
**BE DE ES GB IT NL**

(56) Documents cités:
**FR-A- 2 454 830**
**US-A- 4 026 764**
**US-A- 4 373 025**

**M. DIXON et al.: "Enzymes", 2ième édition, 1972, pages 294,295, Longman;**

**CARBOHYDRATE RESEARCH, vol. 70, 1979, pages 209-216, Elsevier Scientific Publishing Co., Amsterdam, NL; L.W. DONER: "Isomerization of D-fructose by base: liquid-chromatographic evaluation and the isolation of D-psicose"**

**ACTA CHEMICA SCANDINAVICA, vol. B 37, no. 2, 1983, pages 101-108, Acta Chemica Scandinavica; K. BOCK et al.: "Isomerization of D-glucose with glucose-isomerase. A mechanistic study"**

(73) Titulaire: **Roquette Frères**

**F-62136 Lestrem(FR)**

(72) Inventeur: **Huchette, Michel**
**63, rue du Maréchal Joffre**
**F-59660 Merville(FR)**

(74) Mandataire: **Koch, Gustave et al**
**Cabinet PLASSERAUD 84, rue d'Amsterdam**
**F-75009 Paris(FR)**

## Description

L'invention concerne de façon générale l'obtention de dextrose cristallisé à partir de saccharose.

Elle a plus précisément pour objet un procédé pour la transformation quasi-quantitative du saccharose en sirops de glucose et sa cristallisation pour obtenir du dextrose pur.

L'invention a tout particulièrement pour objet un procédé permettant d'obtenir, à partir de saccharose, et ceci de façon essentiellement quantitative, des sirops riches en glucose et du dextrose et, à partir de ces sirops, du dextrose pur ainsi que les utilisations de ces produits.

L'intérêt industriel du dextrose et de ses dérivés, notamment le sorbitol ou D-glucitol, ainsi que de leurs sirops est bien connu, en particulier dans des domaines tels que la pharmacie, la cosmétologie, l'alimentation et la chimie.

Le dextrose pur est traditionnellement obtenu par cristallisation d'un sirop de glucose, lequel constitue le résultat de l'hydrolyse de l'amidon qui est un polymère du glucose et représente le polysaccharide réservoir d'énergie de nombreuses plantes.

Un autre polysaccharide très répandu dans le monde végétal est le saccharose. Celui-ci est présent en grande concentration, en particulier dans la betterave et la canne à sucre.

Il est constitué d'une molécule de glucose et d'une molécule de fructose liées par une liaison béta-1-2. Cette liaison s'hydrolyse facilement sous l'effet des acides ou d'enzymes telles que l'invertase de la levure par exemple, et il est donc facile d'obtenir, à partir de saccharose, des solutions communément désignées sous le nom de "sucre inverti" et qui contiennent du glucose et du fructose en quantités équimoléculaires.

Il est par ailleurs connu de séparer industriellement le glucose et le fructose contenus dans les solutions de sucre inverti, notamment par chromatographie sur résine échangeuse d'ions. Ce procédé permet d'obtenir une fraction riche en fructose, d'une part et une fraction riche en glucose, d'autre part. La fraction riche en fructose peut être concentrée et/ou cristallisée et il en va de même pour la fraction riche en glucose.

Malheureusement, dans ces conditions, la fabrication de dextrose pur et de sirops riches en glucose à partir de saccharose, matière première abondante et à bon marché, est inévitablement liée à la production concomitante et en quantité sensiblement équivalente de fructose.

On conçoit ainsi aisément pourquoi le saccharose n'a pas été utilisé jusqu'à présent comme matière première permettant la production massive de sirops de glucose et de dextrose.

Il est connu également depuis plusieurs années de fabriquer, à partir de solutions riches en glucose, des solutions contenant du fructose. Ces solutions, couramment appelées "sirops isomérisés", sont obtenues par action sur les sirops de glucose d'une enzyme d'isomérisation telle que notamment la glucose isomérase. Le brevet US 4 026 676 décrit ainsi l'isomérisation du glucose en fructose à partir d'un sirop de glucose, par la mise en oeuvre d'une glucose isomérase activée.

Une telle étape d'isomérisation a également déjà été mise en oeuvre à la suite d'un procédé de chromatographie permettant d'obtenir une fraction riche en fructose, d'une part et une fraction riche en glucose, d'autre part. Le brevet US 4 373 025 décrit ainsi un procédé de chromatographie où la fraction riche en glucose est recyclée pour être isomérisée.

Malheureusement, dans ce cas général où l'on part d'un sirop contenant essentiellement du glucose, l'équilibre de la réaction d'isomérisation enzymatique est limité à la formation de quantités sensiblement équimoléculaires de glucose et de fructose.

La solution ainsi obtenue est donc du même type que celle qui est obtenue directement par inversion du saccharose et il est donc nécessaire, pour obtenir du fructose pur, d'avoir recours à un procédé de séparation, comme par exemple une chromatographie du type indiqué plus haut à propos de la séparation industrielle du glucose et du fructose contenus dans les solutions de sucre inverti.

Or, il est du mérite de la demanderesse d'avoir réussi à exploiter avantageusement cet équilibre de la glucose-isomérase dans un procédé permettant d'obtenir, avec un rendement pratiquement quantitatif, des sirops de glucose ou du dextrose à partir de saccharose et, à partir de ces sirops, du dextrose pur.

Cette exploitation avantageuse de l'équilibre est obtenue en isomérisant cette fois, dans un sirop riche en fructose, le fructose en glucose et non le glucose en fructose, comme il a toujours été réalisé dans l'industrie avec la glucose-isomérase.

Plus précisément, l'invention a pour objet un procédé pour la préparation de dextrose pur à partir de saccharose, caractérisé en ce qu'il comprend essentiellement les étapes consistant à:

- hydrolyser une solution de saccharose,
- séparer par chromatographie la solution de sucre inverti obtenue en sirop riche en glucose, d'une part et sirop riche en fructose, d'autre part,
- isomériser enzymatiquement à un pH de 6,9 à 8,5 et, de préférence, de 7,5 à 8,0, le sirop riche en fructose pour le transformer en un sirop enrichi en glucose,
- recycler la fraction isomérisée en amont du dispositif de chromatographie, et

- soumettre le sirop riche en glucose provenant de la chromatographie à une cristallisation pour obtenir du dextrose pur.

Le sirop riche en glucose provenant de la chromatographie peut être utilisé tel quel, ou soumis à cristallisation pour obtenir du dextrose pur.

Avantageusement, les eaux-mères provenant de la cristallisation du dextrose sont elles-mêmes recyclées partiellement ou totalement en amont du dispositif de chromatographie.

L'étape d'isomérisation permet de transformer progressivement tout le fructose en glucose.

Ainsi, grâce au procédé selon l'invention, il est possible de transformer de façon quasi totale le saccharose en glucose et d'obtenir des sirops de glucose ou du dextrose.

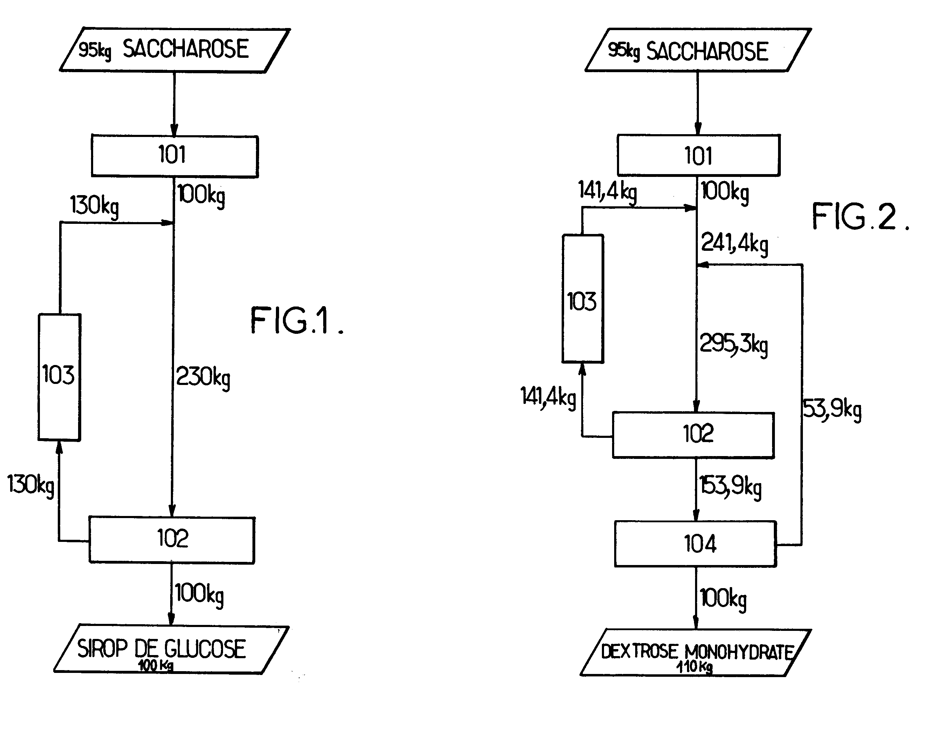

Le procédé conforme à l'invention peut être mis en oeuvre à l'aide de l'installation schématiquement représentée sur la figure 2 de la planche annexée, installation qui est adaptée au procédé et qui comprend essentiellement:

- une enceinte 101 adaptée à l'inversion du saccharose;
- une enceinte 102 de séparation chromatographique du sucre inverti;
- une enceinte 103 munie de glucose-isomérase; et
- une enceinte 104 de cristallisation-séparation du sirop de glucose.

Ne sont pas représentés les divers systèmes de filtration, décoloration, déminéralisation, concentration etc. qu'il est usuel d'adjoindre à ces enceintes et qui sont connus de l'homme du métier.

Des indications plus précises seront données ci-dessous, à titre indicatif, en ce qui concerne la constitution et le fonctionnement de ces différentes enceintes.

L'enceinte 101 reçoit le saccharose qui est-mis en solution aqueuse à une matière sèche comprise généralement entre 30 et 70 % environ et qui y subit l'inversion, notamment par l'action hydrolysante d'invertase de levure, par exemple celle commercialisée par la société GIST BROCADES, Delft, Pays-Bas sous la dénomination MAXINVERT L 10 000, pour obtenir un pouvoir rotatoire spécifique de -19° environ.

Cette enceinte peut être du type continu ou discontinu, mais peut être avantageusement constituée par un réacteur à marche continue contenant l'enzyme sous forme immobilisée ou encore une résine cationique forte sous forme hydrogène.

L'enceinte 102 est constituée d'un système quelconque connu pour son aptitude à séparer en solution le glucose et le fructose. Elle peut avoir un fonctionnement continu ou discontinu. Elle est alimentée par un mélange de sirops provenant des enceintes 101, 103 et 104.

Elle est conçue et exploitée de manière à obtenir d'une part, un sirop riche en glucose contenant de préférence au moins 80 %, et de préférence encore de 88 à 99 % de glucose et d'autre part, un sirop enrichi en fructose.

L'étape de séparation chromatographique peut s'effectuer, de manière connue en soi, de façon discontinue ou continue (lit mobile simulé), sur des adsorbants du type résines cationiques, fortement acides, chargées en ions alcalins ou alcalino-terreux ou du type zéolithes, chargées en ions ammonium, sodium potassium, calcium, strontium ou baryum.

Des exemples de tels procédés de séparation chromatographique sont décrits dans les brevets US 3 044 904, US 3 416 961, US 3 692 582, FR 2 391 754, FR 2 099 336, US 2 985 589, US 4 024 331, US 4 226 977, US 4 293 346, US 4 157 267, US 4 182 633, US 4 332 633, US 4 405 445, US 4 412 866 et US 4 422 881.

Suivant un mode de réalisation préféré, l'étape de séparation chromatographique est effectuée par mise en oeuvre du procédé et de l'appareillage décrits dans le brevet US 4 422 881 et le brevet FR-A-2 454 830 correspondant, dont la Société Demanderesse est la titulaire.

Quel que soit le procédé de séparation chromatographique retenu, on a recours, de préférence à titre d'adsorbant, à une résine cationique forte mise sous forme calcium et ayant un taux de divinylbenzène d'environ 4 à environ 10 %.

L'enceinte 103 est alimentée à partir de l'enceinte 102 en la fraction enrichie en fructose. On procède dans cette enceinte à l'isomérisation du fructose en glucose. L'enzyme utilisée peut être soluble ou insolubilisée. Elle est de préférence insolubilisée sous forme de granulés permettant son utilisation dans un réacteur à effet de piston.

Quel que soit son état, l'enzyme utilisée est une glucose isomérase. Il peut s'agir entre autres de l'une quelconque des glucose isomérases mentionnées ou décrites dans Agr. Biol. Chem. vol. 29, n°12, p 1129-1134 (1965), et vol. 31, n°3, p 284-292 et p 309-313 (1967).

On peut avantageusement utiliser la glucose isomérase immobilisée commercialisée par la société NOVO Bagsvaerd, Danemark, sous la dénomination SWEETZYME type Q.

Les conditions optimales d'utilisation (température, pH, présence d'un sel ou de plusieurs sels métalliques, présence éventuelle d'un agent réducteur) varient sensiblement d'une enzyme à l'autre.

Toutefois, l'isomérisation s'effectue à un pH de 6,9 à 8,5, de préférence de 7,5 à 8,0 et en règle générale à une température de 50 à 70° C, de préférence 60° C et en présence d'un sel de magnésium et d'un agent chimique réducteur tel que le bisulfite de sodium.

L'isomérisation est conduite de façon à obtenir, au sortir de cette enceinte 103, un sirop de fructose isomérisé ayant une teneur en glucose de 25 à 50 %, et de préférence de 35 à 47 % environ. Ce sirop est mélangé, avant d'être chromatographié, au sirop de sucre inverti provenant de l'enceinte 101 et aux eaux-mères provenant de la cristallisation du dextrose dans l'enceinte 104.

L'enceinte 104 comprend un dispositif de cristallisation, de préférence agencé et conduit comme décrit dans le brevet FR 2 555 201 dont la demanderesse est également titulaire, mais aussi un dispositif apte à séparer de leurs eaux-mères les cristaux de dextrose alpha-monohydrate formés lors de cette cristallisation.

Ainsi donc, le procédé selon l'invention permet d'obtenir à partir de saccharose, et éventuellement sans génération de fructose, toute une gamme de produits très riches en glucose dont les applications peuvent être très diverses.

Ce procédé permet d'obtenir du dextrose monohydrate qui répond par exemple à toutes les exigences d'un produit injectable dans le sang mais qui peut également être utilisé dans bien d'autres domaines, chimiques ou alimentaires notamment.

Par déshydratation sous vide à température modérée, on peut transformer ce dextrose monohydrate en la variété anhydre qui est très utilisée dans la déshydratation finale des boissons en poudre selon le procédé d'hydrodessication.

Le dextrose anhydre peut être également obtenu directement par le procédé de l'invention en faisant cristalliser les sirops enrichis en glucose issus de l'étape de chromatographie dans des conditions permettant l'apparition de cette forme cristalline et qui sont généralement une concentration d'environ 85 % et une température finale de cristallisation non inférieure à 50° C.

Par réarrangement moléculaire en milieu anhydre et acide et en présence d'un plastifiant tel que le sorbitol, lui-même obtenu à partir de dextrose, on peut transformer le dextrose en polymère tel que celui commercialisé sous la dénomination POLYDEXTROSE, par la société PFIZER, Terre Haute, Indiana, EUA et qui est un produit peu assimilable.

Par réduction catalytique, et notamment en présence de nickel de Raney ou d'un catalyseur au ruthénium, sous une pression d'hydrogène comprise entre $2.10^6$ et $7.10^6$ Pa environ (20 à 70 kg/cm²), à une température d'environ 100 à 150° C, on peut hydrogéner, après l'avoir remis en solution, le dextrose obtenu par cristallisation des sirops de glucose, et obtenir ainsi un sirop de sorbitol extrêmement pur qui pourra être déshydraté totalement pour former de la poudre de sorbitol.

Ces différentes qualités de sorbitol trouvent application dans les industries chimiques, pharmaceutiques, cosmétologiques et alimentaires notamment. Le sorbitol est également utilisé en papeterie, fonderie et représente la matière première de la synthèse de la vitamine C.

Par oxydation chimique ou biologique, par exemple avec Acetobacter suboxydans ou Aspergillus niger, et en présence de sels minéraux et de matières azotées, on peut transformer le glucose en acide gluconique, lequel à son tour peut être transformé en delta-glucono-lactone utilisée par exemple dans les industries laitières, de panification ou de la viande, et en gluconates alcalins, alcalino-terreux ou métalliques qui trouvent application dans des industries aussi diverses que celles de la pharmacie, de la détergence, des traitements de surface, des bétons et mortiers.

Des oxydations plus complètes peuvent fournir l'acide 2-céto-D-gluconique, matière première de la synthèse de l'acide érythorbique ou l'acide 2,5-dicétogluconique qui semble devenir un intermédiaire intéressant dans la biosynthèse de la vitamine C.

D'autres fermentations oxydatives peuvent fournir les acides citrique, fumarique, itaconique par exemple.

Par fermentation également, avec des bactéries du genre Brevibacterium et Corynebacterium, on peut transformer le glucose en acides aminés, lesquels trouvent emploi dans l'alimentation animale et dans les industries pharmaceutiques et alimentaires et qui sont par exemple l'acide glutamique, la lysine, le tryptophane.

Enfin dextrose et sorbitol peuvent être également utilisés pour en faire des anhydrides, des éthers ou des esters trouvant application dans les industries des matières plastiques ou de la détergence, par exemple.

EXEMPLE 1

Fabrication d'un sirop de glucose avec un rendement quantitatif, à partir de saccharose :

On procède à l'inversion du saccharose dans l'enceinte 101 de la façon suivante.

Celui-ci est dissous dans l'eau à une matière sèche de 50 % et la solution est portée à 60° C. On ajuste le pH à 4,75. On ajoute ensuite la solution enzymatique d'invertase commercialisée par la société GIST-BROCADES, sous la dénomination de MAXINVERT L 10000, à raison de 2 ml par kg de saccharose.

Après 20 heures d'inversion, la composition obtenue est la suivante (analyse par chromatographie liquide haute pression) : 50,5 % de glucose et de 49,5 % de fructose.

Le sirop d'inverti est ensuite mélangé au sirop de fructose isomérisé provenant de l'enceinte 103 dont il sera question ci-après, dans des proportions qui, à l'équilibre du système, sont de 130 kg de matières sèches ayant subi l'isomérisation pour 100 kg de matières sèches provenant de l'inversion.

Le mélange résultant, contenant environ 47 % de glucose et 53 % de fructose est ensuite déminéralisé puis concentré à une matière sèche de 50 % avant d'être acheminé vers l'enceinte de chromatographie 102.

On conduit la chromatographie de façon à extraire le glucose à une richesse de 96 % environ et on l'alimente avec un débit correspondant à 750 kg de matières sèches par m$^3$ d'adsorbant et par jour. L'eau d'élution est alimentée à un débit correspondant à 50 l d'eau pour 100 kg de sirop. Dans ces conditions, la fraction enrichie en fructose titre 9,6 % de glucose.

Après concentration à 45 % de matières sèches, la fraction riche en fructose est dirigée vers l'enceinte 103 pour y subir l'isomérisation enzymatique dans un réacteur de type "piston" garni de glucose isomérase immobilisée, vendue sous la dénomination SWEETZYME type Q par la société NOVO. Cette isomérisation est effectuée en présence de 200 ppm de bisulfite de sodium et de 0,1 g/l de sulfate de magnésium. A l'entrée du réacteur d'isomérisation, le pH est régulé à 7,8 par addition de carbonate de sodium. La température du réacteur est ajustée de façon à obtenir en sortie du réacteur un taux de 45 % de glucose. Elle variera de 56 à 68° C au cours d'une période de trois mois en fonction inverse de l'activité enzymatique immobilisée dans le réacteur. Ce sirop isomérisé est ensuite mélangé au sirop de sucre inverti issu de l'enceinte 101 comme il a déjà été décrit.

Le sirop enrichi en glucose, issu de l'étape de chromatographie 102, est purifié de façon classique par décoloration et/ou déminéralisation, il titre 96 % (sur la base de la matière sèche) de glucose et ne contient pas de polysaccharides.

Les bilans de matières sèches sont résumés sur la figure 1 en regard des différentes opérations unitaires décrites dans cet exemple.

EXEMPLE 2

Fabrication de dextrose monohydrate avec un rendement quantitatif, à partir de saccharose.

Le sirop de glucose obtenu selon l'exemple 1 est particulièrement adapté à la production de dextrose alpha-monohydrate grâce à sa forte richesse en glucose et à sa grande pureté. En outre, l'existence dans le procédé d'une étape de chromatographie permet un recyclage quasi-total des eaux-mères issues de la cristallisation du dextrose.

Pour fabriquer du dextrose monohydrate avec un rendement quasi-total à partir de saccharose, on procède donc comme suit ou de manière équivalente.

Le sirop de glucose issu de l'enceinte de chromatographie 102 est purifié et concentré à une matière sèche de 73 %, propre à permettre la cristallisation du dextrose.

La cristallisation est conduite de la manière décrite dans le brevet FR 2 555 201 et l'essorage de la masse cristallisée fournit du dextrose monohydrate d'une pureté supérieure à 99,5 %. Ce dextrose est obtenu avec un rendement de cristallisation, exprimé sur la matière sèche, de 65 % et dans ces conditions, les eaux-mères totales (eaux-mères vraies plus eaux de lavage des cristaux) qui sont recyclées en tête de chromatographie ont une richesse en glucose de 88,5 %.

Dans les conditions d'équilibre du procédé, les bilans en matière sèche, par opération unitaire, s'établissent comme montré à la figure 2.

**Revendications**

1. Procédé pour la préparation de dextrose pur à partir de saccharose, caractérisé en ce qu'il comprend essentiellement les étapes consistant à :
   - hydrolyser (101) une solution de saccharose,
   - séparer par chromatographie (102) la solution de sucre inverti obtenue en sirop riche en glucose, d'une part et sirop riche en fructose, d'autre part,
   - isomériser enzymatiquement (103) à un pH de 6,9 à 8,5 et, de préférence, de 7,5 à 8,0, le sirop riche en fructose pour le transformer en un sirop enrichi en glucose,
   - recycler la fraction isomérisée en amont du dispositif de chromatographie (102), et
   - soumettre le sirop riche en glucose provenant de la chromatographie (102) à une cristallisation (104) pour obtenir du dextrose pur.

2. Procédé selon la revendication 1, caractérisé en ce que les eaux-mères provenant de la cristallisation du dextrose (104) sont recyclées, partiellement ou totalement, en amont du dispositif de chromatographie (102).

3. Procédé selon l'une des revendications 1 et 2, caractérisé en ce que l'hydrolyse (101) du saccharose est effectuée par action d'une invertase de levure.

4. Procédé selon la revendication 3, caractérisé en ce que l'hydrolyse est effectuée dans un réacteur (101) à marche continue contenant l'enzyme sous forme immobilisée.

5. Procédé selon l'une des revendications 1 à 4, caractérisé en ce que la séparation chromatographique (102) est effectuée au moyen d'une résine cationique forte mise sous forme calcium et ayant un taux de divinylbenzène de 4 à 10% environ.

6. Procédé selon l'une des revendications 1 à 5, caractérisé en ce que l'isomérisation enzymatique (103) est effectuée au moyen d'une glucose-isomérase insolubilisée sous forme de granulés.

7. Procédé selon l'une des revendications 1 à 6, caractérisé en ce que l'isomérisation enzymatique (103) est effectuée à une température de 50 à 70°C et en présence d'un sel de magnésium et d'un agent chimique réducteur tel que le bisulfite de sodium.

8. Installation pour la fabrication de dextrose pur à partir de saccharose, adaptée pour le procédé selon l'une des revendications 1 à 7, caractérisée en ce qu'elle comprend essentiellement:
    - une enceinte (101) adaptée à l'inversion du saccharose;
    - une enceinte (102) de séparation chromatographique du sucre inverti;
    - une enceinte (103) munie de glucose-isomérase; et
    - une enceinte (104) de cristallisation-séparation du sirop de glucose.

## Claims

1. A method for the preparation of pure dextrose from saccharose, characterised in that it essentially comprises the stages consisting of:
    - hydrolysing (101) a solution of saccharose,
    - separating the inverted sugar solution obtained by chromatography (102) into a syrup rich in glucose and a syrup rich in fructose,
    - enzymically isomerising (103) the fructose-rich syrup at a pH of 6.9 to 8.5 and preferably 7.5 to 8.0 in order to convert it into a glucose-enriched syrup,
    - recycling the isomerised fraction upstream of the chromatography device (102), and
    - subjecting the glucose-rich syrup from chromatography (102) to crystallisation (104) to obtain pure dextrose.

2. A method according to claim 1, characterised in that the mother liquors from dextrose crystallisation (104) are wholly or partly recycled upstream of the chromatography device (102).

3. A method according to one of claims 1 and 2, characterised in that hydrolysis (101) of the saccharose is effected using a yeast invertase.

4. A method according to claim 3, characterised in that the hydrolysis is performed in a continuous reactor (101) containing the enzyme in an immobilised form.

5. A method according to one of claims 1 to 4, characterised in that the chromatographic separation (102) is effected using a strong cationic resin in its calcium form and having a divinyl benzene content of approximately 4 to 10%.

6. A method according to one of claims 1 to 5, characterised in that the enzyme isomerisation (103) is effected by means of a glucose-isomerase which is made insoluble in the form of granules.

7. A method according to one of claims 1 to 6, characterised in that the enzyme isomerisation (103) is effected at a temperature of 50 to 70°C in the presence of a magnesium salt and a chemical reducing agent such as sodium bisulphite.

8. An installation for the manufacture of pure dextrose from saccharose suitable for the process according to one of claims 1 to 7 characterised in that it essentially comprises:

    - an enclosure (101) suitable for inversion of the saccharose,
    - an enclosure (102) for chromatographic separation of the invert sugar,
    - an enclosure (103) containing glucose-isomerase, and
    - an enclosure (104) for crystallisation/separation of the glucose syrup.

## Patentansprüche

1. Verfahren zur Herstellung von reiner Dextrose aus Saccharose, dadurch gekennzeichnet, daß es im wesentlichen folgende Schritte aufweist:
    - Hydrolysieren (101) einer Saccharose-Lösung,

- Trennen der erhaltenen Invertzucker-Lösung durch Chromatographie (102) in einen an Glukose reichen Zuckerdicksaft, einerseits, und einen an Fruktose reichen Zuckerdicksaft, andererseits,
- enzymatisches Isomerisieren (103) des an Fruktose reichen Zuckerdicksafts auf einen pH-Wert zwischen 6,9 und 8,5, und vorzugsweise zwischen 7,5 und 8,0, um ihn in einen mit Glukose angereicherten Zuckerdicksaft umzuwandeln,
- Wiedereinbringen des isomerisierten Teils vor der Chromatographie-Vorrichtung (102) und
- Auskristallisieren (104) des von der Chromatographie (102) kommenden, an Glukose reichen Zuckerdicksafts zum Erhalten reiner Dextrose.

2. Verfahren nach Anspruch 1, dadurch gekennzeichnet, daß der von der Auskristallisierung der Dextrose (104) kommende Muttersirup teilweise oder vollständig oberhalb der Chromatographie-Vorrichtung (102) wieder eingebracht wird.

3. Verfahren nach Anspruch 1 oder 2, dadurch gekennzeichnet, daß die Hydrolyse (101) der Saccharose mittels einer Hefeinvertase durchgeführt wird.

4. Verfahren nach Anspruch 3, dadurch gekennzeichnet, daß die Hydrolyse in einem ständig laufenden Reaktor (101) durchgeführt wird, der das Enzym in immobilisierter Form enthält.

5. Verfahren nach einem der Ansprüche 1 bis 4, dadurch gekennzeichnet, daß die chromatographische Trennung (102) mittels eines in die Kalziumform gebrachten und einen Divinylbenzengehalt von etwa 4 bis 10% aufweisenden stark kationischen Harzes erfolgt.

6. Verfahren nach einem der Ansprüche 1 bis 5, dadurch gekennzeichnet, daß die enzymatische Isomerisation (103) mittels einer als Granulat dargereichten, unlösbargemachten Glukose-Isomerase erfolgt.

7. Verfahren nach einem der Ansprüche 1 bis 6, dadurch gekennzeichnet, daß die enzymatische Isomerisation (103) bei einer Temperatur zwischen 50 und 70°C und unter Verwendung eines Magnesiumsalzes und eines chemischen Reduktionsmittels, wie das Natriumbisulfit, erfolgt.

8. Vorrichtung zum Herstellen reiner Dextrose aus Saccharose, die für das Verfahren nach einem der Ansprüche 1 bis 7 ausgelegt ist, dadurch gekennzeichnet, daß sie im wesentlichen

- eine Vorrichtung (101), die für die Inversion der Saccharose geeignet ist,
- eine Vorrichtung (102) zur chromatographischen Trennung des Invertzuckers,
- eine Glukose-Isomerase enthaltende Vorrichtung (103) und
- eine Vorrichtung (104) zum Auskristallisieren/Trennen des Glukosedicksaftes aufweist.

FIG.1.

FIG.2.